(19) Europäisches Patentamt
European Patent Office
Office européen des brevets

(11) EP 2 083 050 A1

(12) EUROPEAN PATENT APPLICATION

(43) Date of publication:
**29.07.2009 Bulletin 2009/31**

(51) Int Cl.:
***C08L 95/00*** *(2006.01)* ***C07C 219/04*** *(2006.01)*

(21) Application number: **08001108.3**

(22) Date of filing: **22.01.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Cognis IP Management GmbH**
**49589 Düsseldorf (DE)**

(72) Inventors:
- **Valls, Ramon**
  **E-08022 Barcelona (ES)**
- **Bigorra Llosas, Joaquin**
  **ES 08201 Sabadell (ES)**
- **Escoda Maria**
  **ES - 08960-St. Just Desvern/Barcelona (ES)**

(74) Representative: **Fabry, Bernd**
**Cognis GmbH**
**Postfach 13 01 64**
**40551 Düsseldorf (DE)**

# (54) Compositions for road contruction

(57) Suggested are new aqueous compositions for road construction, comprising
(a) Asphalt or bitumen,
(b) Polymeric emulsifiers, obtainable by esterification of alkanolamines with mixtures of mono- and dicarboxylic acids and/or their quaternisation products, and optionally
(c) Non-ionic, cationic and/or amphoteric co-emulsifiers, and/or
(d) Solvents.

EP 2 083 050 A1

## Description

### Field of the invention

The present invention is related to the area of construction and concerns new asphalt or bitumen emulsions, and the use of new emulsifiers for making said emulsions.

### Background of the invention

Asphalt and bitumen represent dark-coloured, semisolid to hard, fusible, high-molecular hydrocarbon mixtures, obtained as residues during the processing of crude oil. Usually the products represent colloid systems, particularly brines, comprising resin- or coal-like particles showing molecular masses of 300 to 3.000 Dalton ("asphaltenes") suspended in an oily mass ("maltene"). Asphalt or bitumen emulsions are well-known materials, which are useful for road-making and for other purposes. Their advantages are ease of handling because of their lower viscosities than the starting bitumen, and their ability to be applied at lower temperatures in spraying and other techniques. There are no environmental problems because in use the emulsion breaks and the water evaporates to leave a film of asphalt or bitumen where it is required.

With respect to the production of the emulsions, is very important when the emulsion is applied and mixed with fillers and aggregates, to control the velocity that the emulsion breaks (Breaking Index). This Breaking Index depends basically on the chemical composition of emulsifier used.

For characterising the velocity an asphalt or bitumen emulsion breaks a simple test procedure has been established: Under vigorous stirring fillers are added to 100 g of an asphalt or bitumen emulsion until the emulsion breaks and water is separated. The so-called "breaking index" is calculated in the following manner:

$$\text{Breaking Index} = \frac{\text{Amount of filler [\% b.w.]} * 100}{\text{Amount of emulsion}}$$

The higher the filler portion is, the "more slowly" is the emulsion, i.e. the breaking speed is low. For various reasons the production of emulsions showing low breaking speeds is difficult, e. g. due to the fact that the emulsions show a strong tendency to break when mixed with fillers and aggregates. Also the dispersion of the fillers is often insufficient, that means the coverage of the filler surface with the asphalt or bitumen emulsions is not complete, which causes problems in handling the products.

A key issue for producing asphalt and bitumen emulsions exhibiting a slow breaking velocity is the choice of an adequate emulsifier or emulsifier system. From the state of the art are quite a number of different especially cationic and non-ionic emulsifying systems, optionally including polymers are known. Typical examples can be found in the patents EP 1189990 B1, EP 1179570 B1 or EP 1111010 B1 (all Cognis).

Nevertheless, the emulsifiers found in the market still leave space for improvement, especially with respect to lower breaking velocity and higher storage stability of the emulsions. Therefore it has been the object of the present invention to develop new asphalt and bitumen emulsions and suitable emulsifying systems which improve the properties of the respective products known from the state of the art.

### Detailed description of the invention

The present invention refers to aqueous compositions for road construction, comprising

(a) Asphalt or bitumen,
(b) Polymeric emulsifiers, obtainable by esterification of alkanolamines with mixtures of mono- and dicarboxylic acids and/or their quaternisation products, and optionally
(c) Non-ionic, cationic and/or amphoteric co-emulsifiers, and/or
(d) Solvents.

Surprisingly it has been observed that using the polymeric non-ionic and/or cationic polymeric emulsifiers, optionally in combination with additional co-surfactants, asphalt and bitumen emulsions are obtained showing improved breaking velocity, higher breaking indices and better storage stability. Especially, the emulsions do not show any spontaneous breaking caused by vibrations during transportation.

Asphalt and bitumen

In the following, the terms asphalt and bitumen (component a) are used to describe natural or petroleum-derived bitumen including the well-known penetration grade bitumen, blown or oxidised grades and polymer-modified bitumen, for example, modified with styrene-butadiene polymers or ethylene vinyl acetate polymers. Examples are those having a softening point (Ring and Ball) from 20, preferably from about 50 to about 200 °C. Particularly suitable are those showing softening points between about 30 and about 100, especially between about 35 and about 65 and more particularly between about 40 and about60 °C. Also preferred are those starting materials exhibiting an penetration Index (UNE-EN 1426) at 25 °C between about 30 and about 330.

Polymeric alkanolamine esters and their quaternisation products

According to the present invention polymeric alkanolamine esters, their quaternisation products or mixtures of both components obtainable by quaternisation of the polymeric esters in part are useful as emulsifiers (component b) in order to produce homogenous and storage-stable emulsions. The products and their use for the softening of textiles and fibres are disclosed in detail, for example, in EP 0770594 B1 (Henkel); the teaching of this reference is therefore incorporated by reference. More particularly, the polymeric alkanolamine esters and their quaternisation products are obtained by reacting alkanol amines with a mixture of fatty acids and dicarboxylic acids and optionally quaternising the resulting esters - fully or in part - in known manner, optionally after alkoxylation.

According to the present invention, suitable polymeric alkanolamine esters are derived from alkanolamines are derived from amines following general formula **(I)**:

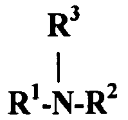

**(I)**

in which $R^1$ represents a hydroxyethyl radical, and $R^2$ and $R^3$ independently from each other stand for hydrogen, methyl or a hydroxyethyl radical. Typical examples are methyldiethanolamin (MDA), monoethanolamine (MES), diethanolamine (DEA) and triethanolamine (TEA). In a preferred embodiment of the present invention, triethanolamine is used as the starting material.

Fatty acids in the context of the invention are understood to be aliphatic carboxylic acids corresponding to formula **(II)**:

$$R^4COOH \qquad (II)$$

in which $R^4CO$ is an aliphatic, linear or branched acyl radical containing 6 to 22 carbon atoms and 0 and/or 1, 2 or 3 double bonds. Typical examples are caproic acid, caprylic acid, 2-ethyl hexanoic acid, capric acid, lauric acid, isotridecanoic acid, myristic acid, palmitic acid, palmitoleic acid, stearic acid, isostearic acid, oleic acid, elaidic acid, petroselic acid, linoleic acid, linolenic acid, elaeostearic acid, arachic acid, gadoleic acid, behenic acid and erucic acid and the technical mixtures thereof obtained, for example, in the pressure hydrolysis of natural fats and oils, in the reduction of aldehydes from Roelen's oxosynthesis or in the dimerization of unsaturated fatty acids. Technical fatty acids containing 12 to 18 carbon atoms, for example, coconut oil, palm oil, palm kernel oil or tallow fatty acids, preferably in hydrogenated or partially hydrogenated form, are preferred.

Dicarboxylic acids suitable for use as starting materials in accordance with the invention correspond to formula **(III):**

$$HOOC\text{-}[X]\text{-}COOH \qquad (III)$$

in which [X] stands for an optionally hydroxysubstituted saturated or unsaturated alk(en)ylene group containing 1 to 10 carbon atoms. Typical examples are succinic acid, maleic acid, glutaric acid, 1,12-dodecanedioic acid and, in particular, adipic acid.

The fatty acids and the dicarboxylic acids may be used in a molar ratio of 1:10 to 10:1. However, it has proved to be of advantage to adjust a molar ratio of 1.5:1 to 2.5:1. The trialkanolamines on the one hand and the acids - i.e. fatty acids and dicarboxylic acids together - on the other hand may be used in a molar ratio of 1:1.3 to 1:2.4. A molar ratio of trialkanolamine to acids of 1:1.4 to 1:1.8 has proved to be optimal. The esterification may be carried out in known manner, for example as described in International patent application WO 91/01295 (Henkel). In one advantageous

embodiment, it is carried out at temperatures between 120 °C and 220 °C, and more particularly from 130 °C to 170 °C under pressures of 0.01 to 1 bar. Suitable catalysts are hypophosphorous acids and alkali metal salts thereof, preferably sodium hypophosphite, which may be used in quantities of 0.01 to 0.1% by weight, and preferably in quantities of 0.05 to 0.07 % b.w. based on the starting materials. In the interests of particularly high colour quality and stability, it has proved to be of advantage to use alkali metal and/or alkaline earth metal borohydrides, for example potassium, magnesium and, in particular, sodium borohydride, as co-catalysts. The co-catalysts are normally used in quantities of 50 to 1000 ppm, and more particularly in quantities of 100 to 500 ppm, again based on the starting materials. Corresponding processes are also the subject of DE 4308792 C1 and DE 4409322 C1 (Henkel) to which reference is hereby specifically made. Mixtures of the fatty acids and dicarboxylic acids may be used or, alternatively, the esterification may be carried out with the two components in successive steps.

Polymeric alkanolamine esters containing polyalkylene oxide may be produced by two methods. First, ethoxylated trialkanolamines may be used. This has the advantage that the distribution of alkylene oxide in the resulting esterquat is substantially the same in regard to the three OH groups of the amine. However, it also has the disadvantage that the esterification reaction is more difficult to carry out on steric grounds. Accordingly, the preferred method is to alkoxylate the ester before quaternisation. This may be done in known manner, i.e. in the presence of basic catalysts and at elevated temperatures. Suitable catalysts are, for example, alkali metal and alkaline earth metal hydroxides and alcoholates, preferably sodium hydroxide, and more preferably, sodium methanolate. The catalysts are normally used in quantities of 0.5 to 5% by weight and preferably in quantities of 1 to 3% by weight, based on the starting materials. Where these catalysts are used, free hydroxyl groups are primarily alkoxylated. However, if calcined hydrotalcites or hydrotalcites hydrophobicized with fatty acids are used as catalysts, the alkylene oxides are also inserted into the ester bonds. This method is preferred where the required alkylene oxide distribution approaches that obtained where alkoxylated trialkanolamines are used. Ethylene and propylene oxide and mixtures thereof (random or block distribution) may be used as alkylene oxides. The reaction is normally carried out at temperatures in the range from 100 °C to 180 °C. The incorporation of, on average, 1 to 10 moles of alkylene oxide per mole of ester increases the hydrophilicity of the esterquat, improves solubility and reduces reactivity to anionic surfactants.

The quaternisation of the fatty acid/dicarboxylic acid trialkanolamine esters in order to produce the quaternised polymeric alkanolamine esters may be carried out also in known manner. Although the reaction with the alkylating agents may also be carried out in the absence of solvents, it is advisable to use at least small quantities of water or lower alcohols, preferably isopropyl alcohol, for the production of concentrates which have a solids content of at least 80% by weight, and more particularly, at least 90% by weight. Suitable alkylating agents are alkyl halides such as, for example, methyl chloride, dialkyl sulfates, such as dimethyl sulfate or diethyl sulphate, for example, or dialkyl carbonates, such as dimethyl carbonate or diethyl carbonate for example. In case it is desired to use fully quaternised polymeric alkanolamine esters the esters and the alkylating agents are normally used in a molar ratio of 1:0.95 to 1:1.05, i.e. in a substantially stoichiometric ratio. In case mixtures of non-quaternised and quaternised products shall be obtained one has to adjust the amount of alkylation agent to the desired degree of quaternisation. Typically, the polymeric emulsifiers according to the present invention represent mixtures of non-quaternised and quaternised polymeric esters in a ratio by weight of about 1:99 to about 99:1, preferably about 25:75 to about 75:25 and more particularly about 40:60 to about 60:40. The reaction temperature is usually in the range from 40 °C to 80 °C, and more particularly, in the range from 50 °C to 60 °C. After the reaction it is advisable to destroy unreacted alkylating agent by addition of, for example, ammonia, an (alkanol)amine, an amino acid or an oligopeptide, as described for example in DE 14026184 A1 (Henkel).

<u>Co-Emulsifiers</u>

In another embodiment of the present invention it has been found advantageous to use the polymeric emulsifiers - quaternised or not - in combination with non-ionic, cationic or amphoteric co-emulsifiers (component c) in order to improve the speed of breaking the emulsion when aggregates are added in the road application. Most preferred is the use of amphoteric co-emulsifiers of the betaine type, like for example alkyl betaines, alkylamido betaines, imidazolins and amphoglycinates.

*<u>Alkyl betaines</u>*

The betaines are known surfactants which are mainly produced by carboxyalkylation, preferably carboxymethylation, of amine compounds. The starting materials are preferably condensed with halocarboxylic acids or salts thereof, more particularly sodium chloroacetate, one mole of salt being formed per mole of betaine. The addition of unsaturated carboxylic acids, such as acrylic acid for example, is also possible. Examples of suitable betaines are the carboxyalkylation products of secondary and, in particular, tertiary amines which correspond to formula **(IV):**

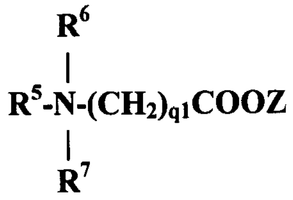

(IV)

where $R^5$ is a an alkyl radical having 6 to 22 carbon atoms, $R^6$ is hydrogen or an alkyl group containing 1 to 4 carbon atoms, $R^7$ is an alkyl group containing 1 to 4 carbon atoms, q1 is a number of 1 to 6 and Z is an alkali and/or alkaline earth metal or ammonium. Typical examples are the carboxymethylation products of hexylmethylamine, hexyldimethylamine, octyldimethylamine, decyldimethylamine, $C_{12/14}$-cocoalkyldimethylamine, myristyldimethylamine, cetyldimethylamine, stearyldimethylamine, stearylethylmethylamine, oleyldimethylamine, $C_{16/18}$-tallowalkyldimethylamine and their technical mixtures, and particularly dodecyl methylamine, dodecyl dimethylamine, dodecyl ethylmethylamine and technical mixtures thereof. The commercially available products include Dehyton® AB (Cognis Deutschland GmbH & Co., KG)

*Alkylamido betaines*

Other suitable betaines are the carboxyalkylation products of amidoamines which correspond to formula **(V):**

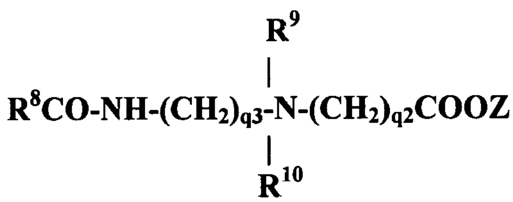

(V)

in which $R^8CO$ is an aliphatic acyl radical having 6 to 22 carbon atoms and 0 or 1 to 3 double bonds, $R^9$ is hydrogen or an alkyl radical having 1 to 4 carbon atoms, $R^{10}$ is an alkyl radical having 1 to 4 carbon atoms, q2 is a number from 1 to 6, q3 is a number from 1 to 3 and Z is an alkali and/or alkaline earth metal or ammonium. Typical examples are reaction products of fatty acids having 6 to 22 carbon atoms, like for example caproic acid, caprylic acid, caprinic acid, lauric acid, myristic acid, palmitic acid, palmoleic acid, stearic acid, isostearic acid, oleic acid, elaidic acid, petroselinic acid, linolic acid linoleic acid, elaeostearic acid, arachidonic acid, gadoleic acid, behenic acid, erucic acid and their technical mixtures with N,N-dimethylaminoethylamine, N,N-dimethylaminopropylamine, N,N-diethylaminoethylamine and N,N-diethylaminopropylamine, which are condensed with sodium chloroacetate. The commercially available products include Dehyton® K and Dehyton® PK (Cognis) as well as Tego®Betaine (Th.Goldschmidt).

*Imidazolines*

Other suitable starting materials for the amphoterics to be used for the purposes of the invention are imidazolines. These substances are also known and may be obtained, for example, by cyclizing condensation of 1 or 2 moles of $C_6$-$C_{22}$ fatty acids with polyfunctional amines, such as for example aminoethyl ethanolamine (AEEA) or diethylenetriamine. The corresponding carboxyalkylation products are mixtures of different open-chain betaines. Typical examples are condensation products of the above- mentioned fatty acids with AEEA, preferably imidazolines based on lauric acid, which are subsequently reacted with sodium chloroacetate. The commercially available products include Dehyton® G (Cognis).

Primary emulsifiers and co-emulsifiers may be applied in ratios by weight of about 50:50 to about 90:10, preferably about 60:40 to about 80:20.

Solvents

It is advisable to use non-polar solvents to obtain a better emulsion, less viscosity and better stability. This optional component (d) may be selected, for example, from mineral oils, hydrocarbons, fatty acid lower alkyl esters such as, for example, the lower alkyl ($C_1$-$C_4$) esters, i.e. methyl, ethyl, propyl and/or butyl esters, of caproic acid, caprylic acid,

2-ethylhexanoic acid, capric acid, lauric acid, isotridecanoic acid, myristic acid, palmitic acid, palmitoleic acid, stearic acid, isostearic acid, oleic acid, elaidic acid, petroselic acid, linoleic acid, linolenic acid, elaeostearic acid, arachic acid, gadoleic acid, behenic acid and erucic acid and technical mixtures thereof. Vegetable triglycerides, for example coconut oil, palm oil, palm kernel oil, sunflower oil, olive oil and the like are also suitable.

Emulsions

In another preferred embodiment of the present invention the compositions comprise

(a) about 10 to about 70, preferably about 20 to about 40 % b.w. asphalt or bitumen,
(b) about 0.15 to about 15, preferably about 0.2 to about 1 % b.w. polymeric emulsifiers, obtainable by esterification of alkanolamines with mixtures of mono- and dicarboxylic acids and/or their quaternisation products,
(c) 0 to about 5, preferably about 1 to about 4 % b.w. non-ionic, cationic and/or amphoteric co-emulsifiers, and/or
(d) 0 to about 40, preferably about 5 to about 30 % b.w. solvents

under the condition that the amounts add with water and optionally additional typical auxiliary agents to 100 % b.w.

Process for obtaining the emulsions

Another object of the present invention is directed to a process for making aqueous compositions for road construction, which is characterised in that aqueous asphalt or bitumen compositions are mixed under vigorous stirring with a working amount of polymeric emulsifiers, obtainable by esterification of alkanolamines with mixtures of mono- and dicarboxylic acids and/or their quaternisation products, and optionally non-ionic, cationic and/or amphoteric co-emulsifiers, and/or solvents in order to obtain homogenous emulsions.

## Industrial application

As explained above, the polymeric alkanolamine esters and their quaternisation products show excellent properties for making low viscous, storage stable asphalt and bitumen emulsions. Another object of the present invention is therefore directed to the use of

(i) polymers obtainable by esterification of alkanolamines with mixtures of mono- and dicarboxylic acids and/or their quaternisation products as emulsifiers for asphalt or bitumen emulsions, and respectively
(ii) polymers obtainable by esterification of alkanolamines with mixtures of mono- and dicarboxylic acids and/or their quaternisation products, in combination with non-ionic, cationic or amphoteric surfactants,

as emulsifiers for asphalt or bitumen emulsions.

Emulsifier concentrates

A final object of the present invention concerns emulsifier concentrates comprising

(a) polymers obtainable by esterification of alkanolamines with mixtures of mono- and dicarboxylic acids and/or their quaternisation products, and
(b) non-ionic, cationic or amphoteric surfactants.

Component (a) and (b) can be present within a ratio by weight of about 50:50 to about 90:10, more particularly about 60:40 to about 80:20. The concentrates are obtainable by blending the two emulsifiers with water at elevated temperatures. Due to the fact that they do not pass a gel phase, high solids contents of 45 to 60 % are achievable.

## Examples

Manufacturing Example M1

POLYESTER AMINE: 567 g (2.1 moles) of tallow fatty acid, 219 g (1.5 moles) of adipic acid and 0.3 g of hypophosphoric acid were introduced into a stirred reactor and heated to 70 °C under a reduced pressure of 20 mbar. 447 g (3 moles) of triethanolamine were then added dropwise in portions and, at the same time, the temperature was increased to 120 °C. After the addition, the reaction mixture was heated to 160 °C, the pressure was reduced to 3 mbar

and the mixture was stirred under those conditions for 2.5 h until the acid value had fallen to below 5 mg KOH/g.

QUATERNIZATION: The mixture was then cooled to 60 °C., the vacuum was broken by introduction of nitrogen and 0.6 g of hydrogen peroxide was added in the form of a 30% by weight aqueous solution. For the quaternisation step, the resulting ester was dissolved in 256 g of isopropyl alcohol and 357 g (2.83 moles) of dimethyl sulfate were added to the resulting solution over a period of 1 hour at such a rate that the temperature did not rise above 65 °C. After the addition, the mixture was stirred for another 2.5 h, the total nitrogen content being regularly checked by sampling. The reaction was terminated when constant total nitrogen content had been reached. A product with a solids content of 85 % b.w. was obtained.

Emulsion Examples 1 to 3

Bitumen emulsions for road application 1, 2, and 3 were prepared using the emulsifiers according to the invention as set out in Manufacturing Example M1. The emulsions were tested according to standard methods used in bitumen emulsions for road application.

The procedure for making the emulsions represents a standard method: Asphalt was heated to 140 °C, solvent (if present) added in a first tank (organic phase) and water at 70 °C in a second tank, emulsifier and possible co-emulsifier were added and the pH adjusted with HCl (water phase). Subsequently organic and aqueous phase were pumped to a special emulsifier device (colloid mill) and the final emulsion cooled down to 40 °C. The compositions and the results are shown in the following Table 1:

Table 1

| Composition and properties of the emulsions [% b.w.] | | | |
|---|---|---|---|
| **Formulation** | **1** | **2** | **3** |
| Asphalt | 63 | 63 | 63 |
| Polyester amine | 0.75 | - | - |
| Quaternised polyester amine | - | 0.75 | 0.35 |
| Emulsifier according to EP 1179570 A1 | - | - | - |
| Co-emulsifier (Cocamidopropyl betaine) | - | - | 0.4 |
| Water | add to 100 | | |
| ***Properties*** | | | |
| Emulsion pH | 2.5 | 2.3 | 2.53 |
| Sieving (NLT-142) | < 0.1 | < 0.1 | < 0.1 |
| Breaking index (filler SIKA) | 40 | 50 | 130 |
| Viscosity Copa Ford (4) 50 °C | 13 sec | 16 sec | 14 sec |

**Claims**

1. Aqueous compositions for road construction, comprising

   (a) Asphalt or bitumen,
   (b) Polymeric emulsifiers, obtainable by esterification of alkanolamines with mixtures of mono- and dicarboxylic acids and/or their quaternisation products, and optionally
   (c) Non-ionic, cationic and/or amphoteric co-emulsifiers, and/or
   (d) Solvents.

2. Compositions according to Claim 1, **characterised in that** said asphalt or bitumen (Component a) is selected from the group consisting of natural or petroleum-derived bitumens including penetration grade bitumens, blown or oxidised grades and polymer-modified bitumens.

3. Compositions according to Claim 1 and/or 2, **characterised in that** said polymeric emulsifiers (component b) are derived from alkanolamines according to general formula **(I),**

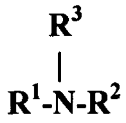

(I)

in which $R^1$ represents a hydroxyethyl radical, and $R^2$ and $R^3$ independently from each other stand for hydrogen, methyl or a hydroxyethyl radical.

**4.** Compositions according to any of the preceding Claims 1 to 3, **characterised in that** said polymeric emulsifiers are derived from triethanolamine.

**5.** Compositions according to any of the preceding Claims 1 to 4, **characterised in that** said polymeric emulsifiers are derived from mixtures of

(i) Monocarboxylic acids according to general formula **(II),**

$$R^4CO\text{-}OH \qquad (II)$$

in which $R^4CO$ stands for a linear or branched acyl radical having 6 to 22 carbon atoms and 0 or 1 to 3 double bonds, and
(ii) Dicarboxylic acids according to general formula **(III),**

$$HOOC\text{-}[X]\text{-}COOH \qquad (III)$$

in which [X] stands for an optionally hydroxysubstituted alk(en)ylene group having 1 to 10 carbon atoms.

**6.** Compositions according to any of the preceding Claims 1 to 5, **characterised in that** said polymeric emulsifiers are derived from mixtures of monocarboxylic acids and dicarboxylic acids in a molar ratio of 1:10 to 10:1.

**7.** Compositions according to any of the preceding Claims 1 to 6, **characterised in that** said polymeric emulsifiers are derived from mixtures of $C_{12}$-$C_{22}$ fatty acids and adipic acid.

**8.** Compositions according to any of the preceding Claims 1 to 7, **characterised in that** said polymeric emulsifiers represent mixtures of non-quaternised and quaternised polymeric esters in a ratio by weight of 1:99 to 99:1.

**9.** Compositions according to any of the preceding Claims 1 to 7, **characterised in that** they comprise as co-emulsifiers (component c) amphoteric surfactants of the betaine or imidazoline type.

**10.** Compositions according to any of the preceding Claims 1 to 9, **characterised in that** they comprise solvents (component d) selected from the group consisting of mineral oils, hydrocarbons, fatty acid lower alkyl esters and vegetable triglycerides

**11.** Compositions according to any of the preceding Claims 1 to 10, **characterised in that** they comprise

(a) 10 to 70 % b.w. asphalt or bitumen,
(b) 0.15 to 15 % b.w. polymeric emulsifiers, obtainable by esterification of alkanolamines with mixtures of mono- and dicarboxylic acids and/or their quaternisation products,
(c) 0 to 5 % b.w. non-ionic, cationic and/or amphoteric co-emulsifiers, and/or
(d) 0 to 40 % b.w. solvents

under the condition that the amounts add with water and optionally additional typical auxiliary agents to 100 % b.w.

**12.** Process for making aqueous compositions for road construction, **characterised in that** aqueous asphalt or bitumen compositions are mixed under vigorous stirring with a working amount of polymeric emulsifiers, obtainable by esterification of alkanolamines with mixtures of mono- and dicarboxylic acids and/or their quaternisation products, and optionally non-ionic, cationic and/or amphoteric co-emulsifiers, and/or solvents in order to obtain homogenous emulsions.

**13.** Use of polymers obtainable by esterification of alkanolamines with mixtures of mono- and dicarboxylic acids and/or their quaternisation products as emulsifiers for asphalt or bitumen emulsions.

**14.** Use of mixtures comprising

(a) Polymers obtainable by esterification of alkanolamines with mixtures of mono- and dicarboxylic acids and/or their quaternisation products, and
(b) Non-ionic, cationic or amphoteric surfactants,

as emulsifiers for asphalt or bitumen emulsions.

**15.** Emulsifier concentrates comprising

(a) Polymers obtainable by esterification of alkanolamines with mixtures of mono- and dicarboxylic acids and/or their quaternisation products, and
(b) Non-ionic, cationic or amphoteric surfactants.

European Patent Office

# EUROPEAN SEARCH REPORT

Application Number
EP 08 00 1108

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,X<br>Y | DE 195 39 846 C1 (HENKEL KGAA [DE]) 21 November 1996 (1996-11-21)<br>* claims 1-11; examples 1-11 *<br>----- | 15<br>1-14 | INV.<br>C08L95/00<br>C07C219/04 |
| X<br>Y | DE 197 51 151 A1 (HENKEL KGAA [DE]) 20 May 1999 (1999-05-20)<br>* claims 1-10; tables 1-3 *<br>----- | 15<br>1-14 | |
| X<br>Y | US 4 859 245 A (SCHILLING PETER [US] ET AL) 22 August 1989 (1989-08-22)<br>* page 3, lines 24-29; claims 1-16 *<br>* column 4, line 65 - column 5, line 2 *<br>* column 6, paragraph 4 - column 7, line 64; examples 1-6 *<br>----- | 1-14<br>1-14 | |
| | | | TECHNICAL FIELDS SEARCHED (IPC)<br>C08L<br>C07C<br>D06M |

The present search report has been drawn up for all claims

1

| Place of search | Date of completion of the search | Examiner |
|---|---|---|
| Munich | 4 June 2008 | olde Scheper, Bernd |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 08 00 1108

This annex lists the patent family members relating to the patent documents cited in the above-mentioned European search report.
The members are as contained in the European Patent Office EDP file on
The European Patent Office is in no way liable for these particulars which are merely given for the purpose of information.

04-06-2008

| Patent document cited in search report | Publication date | Patent family member(s) | Publication date |
|---|---|---|---|
| DE 19539846 C1 | 21-11-1996 | EP 0770594 A1 | 02-05-1997 |
| | | ES 2138778 T3 | 16-01-2000 |
| | | US 5880299 A | 09-03-1999 |
| DE 19751151 A1 | 20-05-1999 | WO 9925797 A1 | 27-05-1999 |
| | | EP 1032626 A1 | 06-09-2000 |
| | | JP 2001523771 T | 27-11-2001 |
| US 4859245 A | 22-08-1989 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1189990 B1
- EP 1179570 B1
- EP 1111010 B1
- EP 0770594 B1
- WO 9101295 A
- DE 4308792 C1
- DE 4409322 C1
- DE 14026184 A1
- EP 1179570 A1